# FASCICULE DE BREVET EUROPEEN

(11) **EP 1 189 877 B1**
(45) Date de publication et mention de la délivrance du brevet: **19.04.2006**
(21) Numéro de dépôt: 00949627.4
(22) Date de dépôt: 04.07.2000
(51) Int. Cl.: C07C 263/04, C07C 265/12

(54) **PROCEDE DE DESHYDROGENOFLUORATION D'UN FLUORURE DE CARBAMOYLE AROMATIQUE**
VERFAHREN DESHYDROGENOFLUORIERUNG VON AROMITISCHEN CARBAMOYLFLUORIDEN
METHOD FOR DEHYDROGENOFLUORINATION OF AN AROMATIC CARBAMOYL FLUORIDE

(30) Priorité: 05.07.1999 FR 9908647
(43) Date de publication de la demande: 27.03.2002
(73) Titulaire: RHODIA CHIMIE, 92512 Boulogne Billancourt Cedex (FR)
(72) Inventeur: GUIDOT, Gilbert, F-30350 Massanes (FR); ROCHIN, Christophe, Princeton, NJ 08540 (US); SAINT-JALMES, Laurent, F-69330 Meyzieu (FR)
(74) Mandataire: Dutruc-Rosset, Marie-Claude
(86) Numéro de dépôt international: PCT/FR2000/001912
(87) Numéro de publication internationale: WO 2001/002347

(56) Documents cités:
- GB-A- 955 898
- R. APPEL ET AL.: "Die Reaktion von Triphenylphosphin/Tetrachlorkohlenstoff mit Carbamoylhalogeniden" CHEMISCHE BERICHTE, vol. 107, 1974, pages 2671-2674, XP002133040 WEINHEIM DE

## Description

La présente invention a pour objet un procédé permettant le traitement d'un fluorure de carbamoyle aromatique pour conduire à l'isocyanate correspondant.

La présente invention a plus particulièrement pour objet un procédé du type précédent qui permette d'obtenir un bon taux de transformation, un bon rendement de transformation, c'est-à-dire une bonne sélectivité, et un bon de rendement de réaction.

Les fluorures de carbamoyle sont des composés assez rares mais dont l'intérêt s'est vu relancé par le rôle grandissant des dérivés fluorés dans la pharmacologie et dans l'agrochimie. En effet, une des techniques les plus classiques pour synthétiser des dérivés présentant un carbone aliphatique perfluoré, consiste à bloquer les éventuelles anilines sous la forme d'un isocyanate, à chlorer (en général la position dérivé alcoylé au moyen de chlore radicalaire, puis traiter le dérivé chloré obtenu par un milieu fluoré, en général un milieu contenant de l'acide fluorhydrique en phase liquide. Au cours de ce type de procédé, la première réaction qui prend place est l'addition de l'acide fluorhydrique sur la fonction isocyanate pour donner un fluorure de carbamoyle.

Ce fluorure de carbamoyle est très difficile à transformer en isocyanate. Ceci est grand dommage car l'isocyanate est un intermédiaire très réactif permettant de nombreuses synthèses et en particulier permettant une libération facile de l'aniline correspondante.

La première partie des réactions est décrite dans différents documents et en particulier dans le document EP-A 152 310 et le document EP-A 129 214. En ce qui concerne le passage du fluorure de carbamoyle à l'isocyanate, le brevet britannique n° 955 898, publié le 22 avril 1964, au nom de la société BAYER indique la possibilité de réaliser cette réaction, notamment à l'exemple 1. Toutefois, la technique utilisée conduit à un faible rendement (35%), la réaction étant, semble-t-il, très difficile et conduisant notamment à des produits lourds et qualifiés de résines.

C'est pourquoi un des buts de la présente invention est de fournir un procédé permettant le passage de fluorure de carbamoyle à une fonction isocyanate dans des conditions opératoires aisées à mettre en oeuvre et permettant de bons rendements de réaction, ainsi qu'une bonne sélectivité.

Une des principales difficultés rencontrées au cours de l'étude qui a mené à la présente invention réside dans la très grande réactivité du fluorure de carbamoyle vis-à-vis de lui-même ou vis-à-vis de noyaux aromatiques riches.

Ces buts et d'autres, qui apparaîtront, par la suite sont atteints au moyen d'un procédé de déshydrofluoration permettant de passer d'un fluorure de carbamoyle aromatique à un isocyanate, caractérisé par le fait qu'il comprend l'addition progressive du fluorure de carbamoyle à l'état dissous ou finement dispersé dans un solvant, dans un pied de solvant à une température au moins égale à 80°C, avantageusement au moins égale à 90°C.

Selon la présente invention, on a pu ainsi démontrer que le caractère finement dispersé ou le caractère dissous du fluorure de carbamoyle à une température où il est réactif, jouait un rôle clé dans l'obtention avec un bon rendement de l'isocyanate.

On mènera de préférence la réaction à une température au plus égale à 150°C.

Ledit solvant présente un point d'ébullition (commençant en cas de mélange) avantageusement d'au moins 100°C, plus préférentiellement d'au moins 120°C.

Il est préférable de s'arranger pour que la pression dans le réacteur soit telle que le solvant soit en ébullition (c'est-à-dire dans la très grande majorité des cas à reflux). On choisira ainsi une pression supérieure à la pression atmosphérique si le solvant présente un point d'ébullition plus bas que la température à laquelle on désire travailler, et une pression inférieure à la pression atmosphérique lorsque le solvant aura un point d'ébullition plus élevé que la température à laquelle on désire travailler.

Selon une des mises en oeuvre préférées de la présente invention, le solvant, qui peut d'ailleurs être un mélange de solvants, est choisi parmi ceux qui sont miscibles avec l'acide fluorhydrique.

Cette miscibilité peut être partielle ou totale, mais il est préférable que la miscibilité soit telle que le solvant, ou le mélange de solvants, choisi présente une capacité à dissoudre l'acide fluorhydrique au moins égale à 5% en volume, de préférence au moins égale à 10% en volume. Cette solubilité présente un intérêt considérable, car cette miscibilité du solvant avec l'acide fluorhydrique permet d'utiliser ce dernier comme tiers-solvant facilitant la solubilisation du fluorure de carbamoyle dans le mélange réactionnel.

Aussi, selon un mode préféré de la présente invention, on utilise l'acide fluorhydrique pour faciliter l'introduction du fluorure de carbamoyle dans le mélange réactionnel. Cette introduction peut être faite à basse température ou être introduite à haute température. Au cours du chauffage ou au cours de l'introduction, lorsque le fluorure de carbamoyle est introduit dans un pied de solvant, l'acide fluorhydrique aidant à la solubilisation est éliminé, mais en s'éliminant, il laisse le fluorure de carbamoyle sous une forme soit très divisée, soit même dissoute au sein du solvant utilisé.

Selon une mise en oeuvre préférée, lors de l'introduction du fluorure de carbamoyle, le rapport entre l'acide fluorhydrique et le fluorure de carbamoyle (rapport HF sur fluorure de carbamoyle) est au moins égal à 2, avantageusement à 3, de préférence à 4.

Selon une des mises en oeuvre préférées de la présente invention, l'addition de fluorure de carbamoyle est réalisée sous la forme d'une solution de ce dernier dans de l'acide fluorhydrique, en respectant les rapports indiqués ci-dessus.

Les meilleurs résultats obtenus correspondent à l'addition d'une solution de fluorure de carbamoyle sur un pied de solvant se trouvant à la température de réaction.

Cette solution est avantageusement une solution dans l'acide fluorhydrique comme indiqué précédemment.

Dans le cas d'une addition sur un pied de solvant, l'addition doit être menée de manière à contrôler le rapport entre l'acide fluorhydrique présent dans le milieu réactionnel, dans ce cas particulier y compris celui additionné plus exactement en équilibre avec le fluorure de carbamoyle et le substrat.

En d'autres termes, le rapport de l'acide fluorhydrique [le libre et celui additionné sur une fonction isocyanate (c'est-à-dire sous la forme de fluorure de carbamoyle)] et les fonctions isocyanate réelles ou masquées sous la forme de fluorure de carbamoyle, est avantageusement au plus égal à 5, de préférence au plus égal à 0,3, plus préférentiellement au plus égal 0,1. Cette condition implique une addition relativement lente du fluorure de carbamoyle.

Selon la présente invention, il est particulièrement avantageux d'éviter la présence d'impuretés ayant un chlore en position benzylique, car dans le cadre de cette réaction, ces impuretés semblent très réactives et détruiraient un certain nombre de substrats ou de composés en dérivant.

A titre indicatif, il est préférable que le nombre de molécules porteuses de chlore en fonction benzylique soit au plus égal à 0,5 à 5%, avantageusement à 2%, de préférence à 1% des fluorures de carbamoyle à traiter.

Les substrats les plus adaptés à la présente invention sont des fluorures de carbamoyle qui comportent un carbone aliphatique d'hybridation sp³ porteur d'au moins deux fluors. Ce carbone aliphatique est en général benzylique, c'est-à-dire qu'il est directement rattaché à un noyau aromatique. Toutefois il peut être rattaché au noyau aromatique par l'intermédiaire d'un chalcogène (notamment oxygène).

La présente invention est particulièrement adaptée au cas où ledit noyau aromatique est celui portant l'azote de la fonction carbamoyle.

Un tel substrat peut comporter plusieurs de ces carbones aliphatiques porteurs d'au moins deux fluors.
Ainsi le substrat répond avantageusement à la formule :

(R)ₘ - Ar(-(CX₂)ₚ-GEA)-NH-CO-F

où :
□ Ar signifie un reste aromatique présentant avantageusement au moins l'une, de préférence deux, plus préférentiellement trois des caractéristiques suivantes :
   - le reste est mononucléaire, c'est-à-dire ne comporte qu'un noyau ;
   - le reste est avantageusement homocyclique ;
   - le reste est à six chaînons ;
□ les X semblables ou différents représentent un fluor ou un radical de formule CₙF₂ₙ₊₁ avec n entier au plus égal à 5 de préférence à 2 ;
□ p représente un entier au plus égal à 2 ;
□ GEA représente un groupe hydrocarboné, un groupe électroattracteur dont les éventuelles fonctions sont inertes dans les conditions de la réaction, avantageusement fluor ou un reste perfluoré de formule CₙF₂ₙ₊₁ avec un entier au plus égale à 8, avantageusement à 5.
   Le nombre total de carbone de -(CX₂)ₚ-GEA est avantageusement compris entre 1 et 15, de préférence entre 1 et 10.
□ m est 0 ou un entier choisi dans l'intervalle fermé (c'est-à-dire comprenant les bornes) 1 à 4 ;
□ R est un substituant inerte dans les conditions opératoires, avantageusement choisi parmi les halogènes avantageusement légers (c'est à dire chlore et fluor) et les radicaux hydrocarbonés, de préférence alcoyle, aryle, alcoylchalcogényle (tel que alcoyloxyle), arylchalcogényle (tel que aryloxyle). Le composé substrat peut notamment être de formule (les substituants éventuels inertes dans les conditions réactionnelles ne sont pas figurés) :

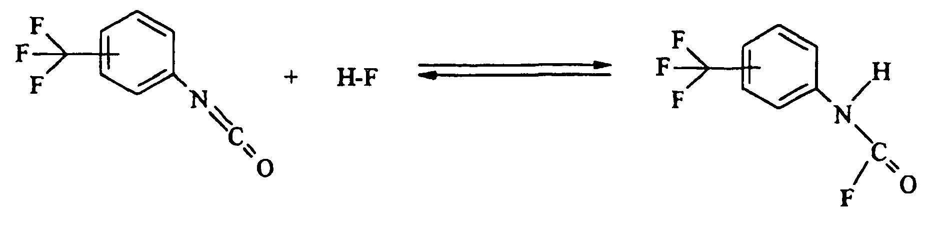

Avantageusement, R est un radical hydrocarboné aryle ou alcoyle, avantageusement d'au plus 10 atomes de carbone, de préférence d'au plus 5 atomes de carbone, R peut également être des fonctions carboxyle, des nitriles, des cétones et des fluorocarbonyles.

Les substituants préférés sont soit rien, c'est-à-dire de l'hydrogène, soit des aryles ou des alcoyles ou des alcoyloxyles.

R ou du moins l'un des R peut également être un groupement comprenant un carbone porteur de deux fluors au moins, de formule CX₂ₚ-GEA comme précédemment.

Les exemples suivants illustrent l'invention.

### Exemple 1

### Elimination de l'acide fluorhydrique dans un solvant susceptible de dissoudre le fluorure de carbamoyle

Dans un réacteur capable de supporter l'acide fluorhydrique en Téflon de 180 ml, chauffé par bain d'huile régulé et sous agitation magnétique, on charge du fluorure de carbamoyle solide (0,1 mol). Puis du trichlorobenzène (chloré en position 1, 3, 4) en quantité de 100,4 g est chargé.

On obtient une suspension facilement à chauffer. On chauffe progressivement à 80°C, le milieu devient homogène et translucide, jaune orangé. Un dégagement gazeux commence aux alentours de 100°C et devient important à partir de 105°C. La température est montée à 125°C et chauffée pendant 7 h. Après refroidissement, on récupère une masse correspondant à un rendement isolé de 80%.

Le composé est ensuite soumis à une distillation et l'on récupère un rendement de 73% en solution dans le trichlorobenzène, quelques résines restant dans le culot de distillation.

### Exemple 2

Différents essais ont été faits en utilisant le monochlorobenzène comme solvant selon divers modes opératoires. Le mode opératoire le plus couramment utilisé est le suivant :

### A) préparation de la solution de fluorure de carbamoyle

On introduit dans un réacteur de l'HF à une température de -5°C. On introduit alors à la température de fluoration désirée, le trichlorométhyle phénylisocyanate que l'on désire transformer, la fluoration dure 1h30, sauf indiquée différemment dans le tableau subséquent, et une opération de finition est menée à la température indiquée dans le tableau pendant 4 h. On observera que lorsque toutes les choses sont égales par ailleurs sauf la température de finition, les rendements sont meilleurs lorsque la température de finition est menée à une température relativement élevée, c'est-à-dire aux alentours de 20-25°C. Ce phénomène montre l'influence extrêmement néfaste de faibles quantités de dérivés restant chlorés une fois en position benzylique.

### B) Rétrogradation en isocyanate

La solution de fluorure de carbamoyle ayant subi ou non une élimination de l'HF préalable, est introduite dans un réacteur dans les conditions spécifiées dans le tableau ci-après. Les conditions de chauffe de pression et de température sont également spécifiées dans le tableau. Les résultats sont rassemblés dans le tableau suivant :

| **n° du test** | **HF 07** | **HF 08** | **HF 09** | **HF 10** | **HF 11** | **HF 12** | **HF 13** | **HF 14** | **HF 15** | HF 16 |
|---|---|---|---|---|---|---|---|---|---|---|
| Température de fluoration | -5°C | -5°C | -5°C | 2°C | 2°C | 2°C | 2°C | -5°C | -5°C | -5°C |
| Température de finition | 2°C | 2°C | 20°C | 20°C | 20°C | 20°C | 20°C | 20°C | 25°C | 25°C |
| Elimination HF préalable | Non | Oui | Non | Oui | Non | Non | Non | Non | Oui | Oui |
| HF libéré au cours de la distillation-réaction | 10 | 10 | 10 | 4,99 | 4,93 | 5,72 | 9,87 | 8,93 | 3,72 | 3,34 |
| Pression | Atmosphérique | Atmosphérique | Atmosphérique | Vide (300 mm) | Vide (300 mm) | Atmosphérique | Atmosphérique | Atmosphérique | 2 atm | 2 atm |
| Lot ou Semi-continu | Semi-continu | Lot | Semi-continu | Lot | Semi-continu | Semi-continu | Semi-continu | Semi-continu | Semi-continu | Semi-continu |
| Coulée sur MCB (durée) | 1h45 | | 0 4h | | 0 2h | 4h | 4h | 4h | 4h | 4h |
| Temp, initiale-finale | 130-130°C | 20-130°C | 130-130°C | 48-100°C | 100-100°C | 130-130°C | 130-130°C | 130-130°C | 140-150°C | 130-130°C |
| | (reflux) | (reflux) | (reflux) | (reflux) | (reflux) | (reflux) | (reflux) | (reflux) | (reflux) | (reflux) |
| Durée de la montée | Néant | 3h | Néant | 1h | Néant | néant | néant | néant | néant | néant |
| Durée du maintien | 2h30 | 3h | 2h | | 0 | 0 2h | 2 h | 2h | 2h | 2h |
| Mole initiale | 1,67 | 1,67 | 1,67 | 1,66 | 1,66 | 1,63 | 1,67 | 1,86 | 1,86 | 1,67 |
| Mole avant distillation | 1,18 | 1,35 | 1,51 | 1,42 | 1,35 | 1,35 | 1,55 | 1,46 | 1,43 | 1,36 |
| Dégagement COF2 | 0,05 | 0,05 | 0,05 | 0,09 | 0,09 | 0,08 | 0,06 | 0,7 | 0,11 | 0,1 |
| Rdt | 70,66% | 80,84% | 90,42% | 85,54% | 81,33% | 82,82% | 92,81% | 78,49% | 76,88% | 81,44% |
| Rdt de distillation | 99,00% | 84,00% | 93,00% | 100,00% | 85,00% | 97,00% | 90,00% | 100,00% | 104,00% | 95,00% |
| Rdt total après distillation | 70,00% | 68,00% | 84,00% | 85,00% | 76,00% | 80,00% | 83,00% | 79,00% | 80,00% | 78,00% |
| Répartition des composés légers dans le mélange réactionnel final avant distillation | | | | | | | | | | |
| Fluorure de carbamoyle | | | 0,70% | 3,00% | 1,43% | 0,47% | 0,34% | 2,30% | 1% | 4,50% |
| Dimère | | | 0,90% | 2,50% | 1,60% | 1,10% | 0,98% | 0,80% | 1% | 0,90% |
| Urée | | | 1,10% | 2,00% | 1,00% | 2,50% | 1,60% | 1,00% | 1% | 1,30% |
| Biuret | | | 6,60% | 12% | 12,40% | 10,00% | 7,10% | 10,50% | 16% | 15,10% |
| Isocyanate | 82,93% | 77,59% | 90,70% | 80,50% | 83,57% | 85,93% | 89,98% | 85,40% | 81% | 78,20% |
| Rapport isocyanate/biuret | | | 13,74242424 | 6,708333333 | 6,739516129 | 8,593 | 12,67323944 | 8,133333333 | 5,0625 | 5,178807947 |

### Commentaire sur les résultats ci-dessus

Les tests HF 07 et HF 08 quoique pas vraiment mauvais, donnent un rendement relativement médiocre en isocyanate final. L'explication de ce phénomène est liée à la faible température de finition qui laisse une proportion non négligeable supérieure à 2 ou 3% en monochloro-difluorométhylphényle.

Une autre raison pour laquelle l'essai HF 07 est médiocre, réside dans la rapidité de la coulée qui ne dure que 1h45 alors que dans les autres cas elle dure significativement plus longtemps.

Pour les essais HF 07 et HF 08, les données sur les impuretés manquent, aussi ne peut-on pas en conclure beaucoup plus. Pour les tests pour lesquels cette donnée est présente, on peut utiliser le rapport isocyanate/biuret comme indice de la sélectivité de la réaction.

La réaction HF 09 donne de très bons résultats, tant au niveau du rendement global, qu'au niveau de la pureté du produit obtenu. Cela démontre l'intérêt d'une part d'ajouter le fluorure de carbamoyle sous une forme dissoute dans l'acide fluorhydrique, et d'autre part de travailler avec un rapport HF/fluorure de carbamoyle élevé. Dans ce cas-là, le rapport HF/isocyanate est de 10, ce qui correspond au rapport acide fluorhydrique/fluorure de carbamoyle de 9. Les résultats des essais HF 10 et HF 11 sous vide sont certes bons, mais un peu décevants compte tenu du fait d'avoir travaillé sous vide. Cependant, la vitesse d'addition ou la vitesse de montée en température était sans doute un peu trop élevée.

L'essai HF 12 est très similaire à l'essai HF 09, mais la différence avec celui-ci vient du fait de la moins grande teneur d'acide fluorhydrique dans le mélange, plus exactement la solution additionnée.

Dans le test HF 13, on retrouve des niveaux d'acide fluorhydrique élevés, et donc une sélectivité de la réaction bien meilleure.

Les essais HF 15 et HF 16 montrent l'effet de l'augmentation de la pression, lequel n'est pas extrêmement favorable.

## Revendications

1. Procédé de déshydrogénofluoration permettant de passer d'un fluorure de carbamoyle aromatique à l'isocyanate correspondant, **caractérisé par le fait qu'**il comprend l'addition progressive du fluorure de carbamoyle à l'état dissous ou finement dispersé dans un solvant, dans un pied de solvant à une température au moins égale à 80°C, avantageusement au moins égale à 90°C.

2. Procédé selon la revendication 1, **caractérisé par le fait que** ladite température de réaction est au plus égale à 150°C.

3. Procédé selon les revendications 1 et 2, **caractérisé par le fait que** ledit solvant présente un point d'ébullition d'au moins 100°C, avantageusement 120°C.

4. Procédé selon les revendications 1 à 3, **caractérisé par le fait que** la réaction est menée à une pression telle que, à la température de réaction, le solvant soit en ébullition.

5. Procédé selon les revendications 1 à 4, **caractérisé par le fait que** le solvant est choisi parmi ceux qui sont miscibles avec l'acide fluorhydrique, avantageusement parmi les dérivés aromatiques halogénés non réactifs avec le fluorure de carbamoyle.

6. Procédé selon les revendications 1 à 5, **caractérisé par le fait que** ledit fluorure de carbamoyle est introduit dans le solvant avec de l'acide fluorhydrique, avantageusement sous la forme d'une solution dans de l'acide fluorhydrique anhydre.

7. Procédé selon la revendication 6, **caractérisé par le fait que** le rapport entre l'acide fluorhydrique et le fluorure de carbamoyle (HF/fluorure de carbamoyle) est au moins égal à 2, avantageusement à 3, de préférence à 4.

8. Procédé selon les revendications 1 à 7, **caractérisé par le fait que** l'addition du fluorure de carbamoyle sous la forme d'une solution, a lieu progressivement sur un pied de solvant porté à la température de réaction choisie.

9. Procédé selon les revendications 1 à 8, **caractérisé par le fait que** l'addition est menée à une vitesse telle que le rapport molaire entre acide fluorhydrique et isocyanate (acide HF/isocyanate aromatique) soit toujours inférieur à 0,5, avantageusement à 0,3, de préférence à 0,1.

10. Procédé selon les revendications 1 à 9, **caractérisé par le fait que** le substrat fluorure de carbamoyle comporte un carbone aliphatique c'est-à-dire d'hybridation sp³ porteur d'au moins deux fluors.

11. Procédé selon la revendication 10, **caractérisé par le fait que** ledit carbone aliphatique porteur d'au moins deux fluors est benzylique, c'est-à-dire qu'il est directement rattaché à un noyau aromatique.

12. Procédé selon la revendication 11, **caractérisé par le fait que** ledit noyau aromatique est celui portant l'azote de la fonction carbamoyle.

13. Procédé selon les revendications 1 à 12, **caractérisé par le fait que** le mélange réactionnel comporte moins de 1 % par rapport au fluorure de carbamoyle initial exprimé en mole d'impuretés présentant un chlore en position benzylique.

14. Procédé selon les revendications 1 à 13, **caractérisé par le fait que** substrat répond à la formule :
(R)ₘ - Ar(-(CX₂)ₚ-GEA)-NH-CO-F
où :
□ Ar et un reste aromatique
□ les X semblables ou différents représentent un fluor ou un radical de formule CₙF₂ₙ₊₁ avec n entier au plus égal à 5 de préférence à 2 ;
□ p représente un entier au plus égal à 2 ;
□ GEA représente un groupe hydrocarboné, un groupe électroattracteur dont les éventuelles fonctions sont inertes dans les conditions de la réaction, avantageusement fluor ou un reste pertluoré de formule CₙF₂ₙ₊₁ avec un entier au plus égale à 8, avantageusement à 5.
Le nombre total de carbone de -(CX₂)ₚ-GEA est avantageusement compris entre 1 et 15, de préférence entre 1 et 10.
□ m est 0 ou un entier choisi dans l'intervalle fermé (c'est-à-dire comprenant les bornes) 1 à 4 ;
□ R représente des radicaux semblables ou différents choisis parmi les halogènes avantageusement légers (c'est à dire chlore et fluor) et les radicaux hydrocarbonés, de préférence alcoyle, aryle, alcoylchalcogényle (tel que alcoyloxyle), arylchalcogényle (tel que aryloxyle).

15. Procédé selon la revendication 14 **caractérisé par le fait que** le substrat de départ répond à la formule suivante ;

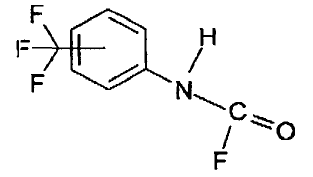

16. Procédé selon les revendications 1 à 15, **caractérisé par le fait que** les solvants sont choisis parmi les chlorobenzénes, avantageusement monochloro, dichloro et trichlorobenzènes.

## Claims

1. A dehydrofluorination process for transforming an aromatic carbamoyl fluoride into the corresponding isocyanate, **characterized in that** it comprises progressively adding carbamoyl fluoride, in the dissolved or finely dispersed state in a solvent, to a seed solvent at a temperature of at least 80°C, advantageously at least 90°C.

2. A process according to claim 1, **characterized in that** said reaction temperature is at most 150°C.

3. A process according to claim 1 or claim 2, **characterized in that** said solvent has a boiling point of at least 100°C, advantageously 120°C.

4. A process according to claims 1 to 3, **characterized in that** the reaction is carried out at a pressure such that the solvent is boiling at the reaction temperature.

5. A process according to claims 1 to 4, **characterized in that** the solvent is selected from those which are miscible with hydrofluoric acid, advantageously from halogenated aromatic derivatives which are not reactive with the carbamoyl fluoride.

6. A process according to claims 1 to 5, **characterized in that** said carbamoyl fluoride is introduced into the solvent with hydrofluoric acid, advantageously in the form of a solution in anhydrous hydrofluoric acid.

7. A process according to claim 6, **characterized in that** the ratio between the hydrofluoric acid and the carbamoyl fluoride (HF/carbamoyl fluoride) is at least 2, advantageously at least 3, preferably at least 4.

8. A process according to claims 1 to 7, **characterized in that** addition of the carbamoyl fluoride in the form of a solution is carried out progressively into a seed solvent heated to the selected reaction temperature.

9. A process according to claims 1 to 8, **characterized in that** addition is carried out at a rate such that the mole ratio between the hydrofluoric acid and the isocyanate (HF acid/aromatic isocyanate) is always less than 0.5, advantageously less than 0.3, preferably less than 0.1.

10. A process according to claims 1 to 9, **characterized in that** the carbamoyl fluoride substrate comprises an aliphatic carbon, i.e. sp³ hybridized, carrying at least two fluorines.

11. A process according to claim 10, **characterized in that** said aliphatic carbon carrying at least two fluorines is benzylic, i.e. directly bonded to an aromatic ring.

12. A process according to claim 11, **characterized in that** said aromatic ring is that carrying the nitrogen of the carbamoyl function.

13. A process according to claims 1 to 12, **characterized in that** the reaction mixture comprises less than 1% with respect to the initial carbamoyl fluoride, expressed as the moles of impurities having a chlorine in the benzyl position.

14. A process according to claims 1 to 13, **characterized in that** the substrate has formula:
(R)ₘ-Ar(-(CX₂)ₚ-GEA)-NH-CO-F
in which:
• the Xs, which may be similar or different, represent a fluorine or a radical with formula CₙF₂ₙ₊₁ where n is an integer of at most 5 and preferably at most 2;
• p represents an integer of at most 2;
• GEA represents a hydrocarbon group, an electro-attractive group any functional groups of which are inert under the reaction conditions, advantageously fluorine or a perfluorinated residue with formula CₙF₂ₙ₊₁ with an integer of at most 8, advantageously at most 5; the total carbon number of -(CX₂)ₚ-GEA is advantageously in the range 1 to 15, preferably in the range 1 to 10;
• m is 0 or an integer in the range 1 to 4 (limits included);
• R represents similar or different radicals selected from halogens, advantageously light halogens (i.e. chlorine and fluorine) and hydrocarbon radicals, preferably alkyl, aryl, alkylchalcogenyl (such as alkyloxyl), arylchalcogenyl (such as aryloxyl).

15. A process according to claim 1, **characterized in that** the starting substrate has the following formula:

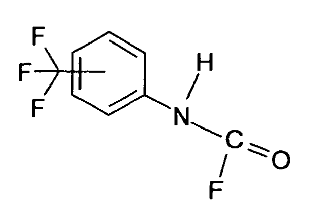

16. A process according to claims 1 to 15, **characterized in that** the solvents are selected from chlorobenzenes, advantageously monochloro-, dichloro- and trichloro-benzenes.

## Patentansprüche

1. Verfahren zur Dehydrogenfluorierung, weiches die Überführung eines aromatischen Carbamoylfluorids zu dem entsprechenden Isocyanat ermöglicht, **dadurch gekennzeichnet, daß** das Verfahren die fortschreitende Zugabe des in einem Lösemittel im gelösten oder feinverteilten Zustand befindlichen Carbamoylfluorids zu einem Lösemittelbodensatz bei einer Temperatur von mindestens 80 °C, vorzugsweise mindestens 90 °C, umfaßt.

2. Verfahren nach Anspruch 1, **dadurch gekennzeichnet, daß** die Reaktionstemperatur höchstens 150 °C beträgt.

3. Verfahren nach Anspruch 1 und 2, **dadurch gekennzeichnet, daß** das Lösemittel einen Siedepunkt von mindestens 100 °C, vorzugsweise 120 °C, aufweist.

4. Verfahren nach den Ansprüchen 1 bis 3, **dadurch gekennzeichnet, daß** die Reaktion bei einem solchen Druck durchgeführt wird, daß bei der Reaktionstemperatur das Lösemittel siedet.

5. Verfahren nach den Ansprüchen 1 bis 4, **dadurch gekennzeichnet, daß** das Lösemittel ausgewählt ist aus solchen Lösemitteln, welche mit Fluorwasserstoff (Fluorwasserstoffsäure) mischbar sind, vorzugsweise aus mit dem Carbamoylfluorid nichtreaktiven, halogenierten aromatischen Derivaten.

6. Verfahren nach den Ansprüchen 1 bis 5, **dadurch gekennzeichnet, daß** das Carbamoylfluorid mit dem Fluorwasserstoff, vorzugsweise in Form einer wäßrigen Lösung in dem wasserfreien Fluorwasserstoff, in das Lösemittel eingetragen wird.

7. Verfahren nach Anspruch 6, **dadurch gekennzeichnet, daß** das Fluorwasserstoff/Carbamoylfluorid-Verhältnis (HF/Carbamoylfluorid-Verhältnis) wenigstens 2, vorzugsweise 3, bevorzugt 4, beträgt.

8. Verfahren nach den Ansprüchen 1 bis 7, **dadurch gekennzeichnet, daß** die Zugabe des Carbamoylfluorids in der Form einer Lösung fortschreitend zu einem Lösemittelbodensatz (Lösemittelsumpf) erfolgt, welcher auf die gewählte Reaktionstemperatur gebracht ist.

9. Verfahren nach den Ansprüchen 1 bis 8, **dadurch gekennzeichnet, daß** die Zugabe mit einer solchen Geschwindigkeit erfolgt, daß das Molverhältnis zwischen Fluorwasserstoff und Isocyanat (Fluorwasserstoffsäure HF/aromatisches Isocyanat) stets kleiner als 0,5, vorzugsweise 0,3, bevorzugt 0,1, ist.

10. Verfahren nach den Ansprüchen 1 bis 9, **dadurch gekennzeichnet, daß** die Carbamoylfluoridausgangsverbindung einen aliphatischen Kohlenstoff umfaßt, d. h. mit einer sp³⁻Hybridisierung, welcher mindestens zwei Fluoratome trägt.

11. Verfahren nach Anspruch 10, **dadurch gekennzeichnet, daß** der aliphatische Kohlenstoff, welcher mindestens zwei Fluoratome trägt, benzylisch ist, d. h. daß er direkt an einen aromatischen Kern gebunden bzw. angeschlossen ist.

12. Verfahren nach Anspruch 11, **dadurch gekennzeichnet, daß** der aromatische Kern den Stickstoff der Carbamoylfunktion trägt.

13. Verfahren nach den Ansprüchen 1 bis 12, **dadurch gekennzeichnet, daß** die Reaktionsmischung, bezogen auf das Anfangscarbamoylfluorid und berechnet als Mol, weniger als 1 % Verunreinigungen mit einem Chlor in der Benzylposition umfaßt.

14. Verfahren nach den Ansprüchen 1 bis 13, **dadurch gekennzeichnet, daß** die Anfangsverbindung der allgemeinen Formel:
(R)ₘ-Ar(-(CX₂)ₚ-GEA)-NH-CO-F
enthält, wobei:
• X, gleich oder verschieden, ein Fluoratom oder einen Rest der Formel CₙF₂ₙ₊₁ darstellt, wobei n eine ganze Zahl von höchstens 5, vorzugsweise 2, ist;
• p eine ganze Zahl von höchstens 2 darstellt;
• GEA eine Kohlenwasserstoffgruppe, eine elektronenziehende Gruppe, deren gegebenenfalls vorhandene funktionelle Gruppen unter den Reaktionsbedingungen inert sind, vorzugsweise Fluor, oder einen perfluorierten Rest der Formel CₙF₂ₙ₊₁, wobei n eine ganze Zahl von höchstens 8, vorzugsweise 5, ist, darstellt;
• die Gesamtanzahl der Kohlenstoffe von -(CX₂)ₚ-GEA vorzugsweise 1 bis 15, bevorzugt 1 bis 10, beträgt;
• m 0 oder eine ganze Zahl in dem geschlossenen Intervall von 1 bis 4 (d. h. einschließlich der Grenzen) ist; und
• R Reste, gleich oder verschieden, darstellt, welche ausgewählt sind aus vorzugsweise leichten Halogenen (d. h. Chlor und Fluor) und Kohlenwasserstoffresten, vorzugsweise Alkyl, Aryl, Alkylchalkogenyl (wie Alkyloxyl) und Arylchalkogenyl (wie Aryloxyl).

15. Verfahren nach Anspruch 1, **dadurch gekennzeichnet, daß** die Anfangsverbindung der folgenden Formel entspricht:

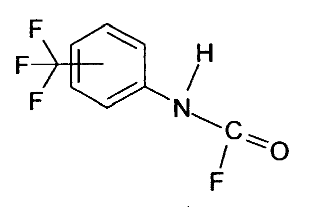

16. Verfahren nach den Ansprüchen 1 bis 15, **dadurch gekennzeichnet, daß** die Lösemittel ausgewählt sind aus Chlorbenzol, vorzugsweise Monochlor-, Dichlor- und Trichlorbenzolen.
